# EUROPEAN PATENT APPLICATION

(11) **EP 3 872 189 A1**
(43) Date of publication of application: **01.09.2021**
(21) Application number: 19875829.4
(22) Date of filing: 21.10.2019
(51) Int. Cl.: C12Q 1/686, C12Q 1/6876

(54) **PROBE HAVING OCTAMINE OR OCTAMINE DERIVATIVE BOUND THERETO, AND USES OF SAME**

(30) Priority: 22.10.2018 KR 20180125973
(71) Applicant: Bioneer Corporation, Daejeon 34302 (KR)
(72) Inventor: PARK, Han-Oh, Sejong 30151 (KR); KWON, Taewoo, Daejeon 34022 (KR); PARK, Sang Ryoung, Daejeon 34048 (KR)
(74) Representative: Mewburn Ellis LLP
(86) International application number: PCT/KR2019/013833
(87) International publication number: WO 2020/085741

(57) **Abstract**

The present invention relates to a probe having octamine or octamine derivative bound thereto in addition to a reporter and a quencher to thus allow the quencher to more effectively suppress light emitted by the reporter, and to uses of the probe. When the probe according to the present invention is utilized, octamine or octamine derivative bound to the probe effectively suppresses the light emitted by the quencher for the reporter, and results in effects such as i) a reduction in base fluorescence, ii) an increase in delta fluorescence, and iii) a decrease in the value of cycle at threshold, thus allowing the probe to be effectively used in a variety of real-time polymerase chain reactions requiring accuracy and sensitivity.

## Description

### [Technical Field]

The present invention relates to a probe to which an octamine or octamine derivative is bound, and the use thereof, and more particularly, to a probe to which a reporter and a quencher are bound and to which an octamine or octamine derivative is further bound to enable the quencher to more effectively suppress light emission by the reporter, and the use thereof.

### [Background Art]

Probe hydrolysis real-time polymerase chain reaction (also called "probe hydrolysis quantitative polymerase chain reaction" and "probe hydrolysis qPCR") is one of test methods that can monitor gene amplification in real time and uses a pair of primers for amplifying a gene having a specific sequence and a probe that complementarily binds to the sequence of the amplification product. In this case, in general, a fluorescent material binds to one end of the probe and a quencher that absorbs light from the fluorescent material binds to the other end thereof. The generation of the fluorescent material binding to the probe is suppressed due to the neighboring quencher.

Meanwhile, a Taq DNA polymerase has 5'→exonuclease activity, whereby the probe bound to the amplification product decomposes into a single nucleotide upon the polymerization reaction. At this time, the fluorescent substance is cleaved from the single nucleotide bound to the probe, thus resulting in generation of fluorescence. Therefore, as the cycle proceeds, the amount of the amplification product increases, more fluorescent substances are separated from the probe, and thus the amount of fluorescence increases.

The degree of inhibition of fluorescence emission varies depending on the structure of the quencher and the type of fluorescent material. Even when the quencher is bound to a probe, emission of fluorescence cannot be completely suppressed, and thus basal fluorescence may occur even in a non-amplified state. When the concentration of the probe is constant, the total amount of fluorescence generated after amplification is also constant. For this reason, the value of delta fluorescence varies depending on the amount of basal fluorescence. Therefore, when the quencher has a weak effect of suppressing light emission, the delta fluorescence amount of fluorescence generated by amplification decreases, the required degree of fluorescence is not sufficiently generated, and thus the accuracy and sensitivity of the real-time polymerase chain reaction may be disadvantageously greatly reduced.

Korean Patent Laid-Open Publication No. 10-2010-0124705 discloses a nucleic acid hybridization method and demonstrates that the disclosed oligonucleotide-oligocation conjugate exhibits strict specificity and very high affinity for a specific target sequence, thus improving the polymerase chain reaction. The patent discloses that the disclosed oligonucleotide-oligocation conjugate as a primer and probe can detect and amplify target nucleic acids. The present invention applies probes with various structures capable of improving the accuracy and sensitivity of real-time polymerase chain reaction by suppressing light emission by quenchers using octamine or octamine derivatives.

Accordingly, as a result of extensive efforts to devise methods to improve the accuracy and sensitivity of the real-time polymerase chain reaction by sufficiently suppressing the fluorescence emission of the reporter by the quencher in the state in which both the reporter and the quencher are bound to the probe, the present inventors found that the effect of suppressing reporter fluorescence emission by the quencher can be improved when the octamine or octamine derivative is bound along with the reporter and quencher to the probe. Based on this finding, the present invention has been completed.

### [Disclosure]

### [Technical Problem]

Therefore, the present invention has been made in view of the above problems, and it is one object of the present invention to provide a novel probe capable of improving the sensitivity of quantitative real-time polymerase chain reaction (qPCR), a kit for quantitative real-time polymerase chain reaction (qPCR) comprising the probe, and a quantitative real-time polymerase chain reaction (qPCR) method using the probe.

### [Technical Solution]

In accordance with one aspect of the present invention, the above and other objects can be accomplished by the provision of a probe to which a reporter, a quencher, and an octamine or octamine derivative are bound.

In accordance with another aspect of the present invention, provided is a method for amplifying a target nucleic acid comprising performing a real-time polymerase chain reaction using the probe.

In accordance with another aspect of the present invention, provided is a kit for real-time polymerase chain reaction comprising the probe.

### [Description of Drawings]

FIG. 1 illustrates a conventional probe (A) having a structure in which a reporter and a quencher are bound to oligonucleotide and probes (B to D) according to various embodiments of the present invention.
FIG. 2 shows comparison in the baseline fluorescence, delta fluorescence and cycle-at-threshold (Ct) values in an amplification experiment of CSF2 gene between a probe to which ethylene propylene octamine is bound, a probe to which ethylene propylene octamine is bound through phosphothioate, and a conventional probe.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one embodiment of the present invention, when ethylene propylene octamine, which is one of octamines, is bound to the end of the probe together with EBQ, which is a quencher, it is possible to reduce basal fluorescence to less than about 50% compared to when only the EBQ quencher is used, by reducing the distance between the quencher and the fluorescent material. Accordingly, it was found that the delta fluorescence increased and the effect of reducing the cycle at threshold (Ct) was obtained.

Therefore, in one aspect, the present invention is directed to a probe to which a reporter, a quencher, and an octamine or octamine derivative are bound.

In the present invention, the probe may be selected from the group consisting of synthetic and artificial deoxyribonucleic acid (DNA), peptide nucleic acid (PNA), and locked nucleic acid (LNA), but is not limited thereto.

In the present invention, the reporter may comprise at least one selected from the group consisting of FAM (6-carboxyfluorescein), TET (5-tetrachlorofluorescein), Texas red, HEX (2',4',5',7',-tetrachloro-6-carboxy-4,7-dichlorofluorescein), Joh, Cy3, Rox, Cy5.5 and CY5, but is not limited thereto.

In the present invention, the quencher may comprise at least one selected from the group consisting of 6-carboxytetramethyl-rhodamine (TAMRA), BHQ1, BHQ2, EBQ and Dabcyl, but is not limited thereto.

In the present invention, the octamine may be ethylene propylene octamine or duodecamine phosphoamidite, but is not limited thereto.

In one embodiment of the present invention, the octamine may be ethylene propylene octamine (C₇₆H₉₇F₂₄N₁₀O₁₃P, molecular weight 1845.58), which is represented by the following Formula 1:

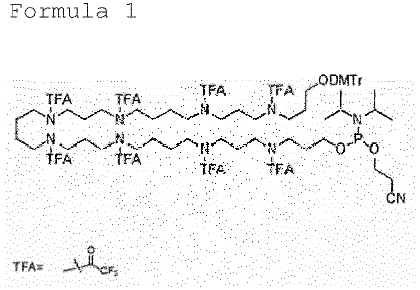

Meanwhile, the structure in which ethylene propylene octamine is bound to the probe is represented by the following Formula 2 (C₃₀H₆₈N₈O₂, molecular weight: 572.91) or Formula 3 (C₃₀H₆₈N₈O₄P-, molecular weight: 635.89) .

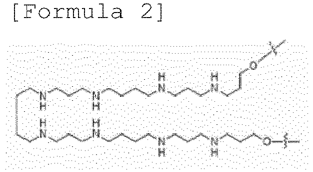

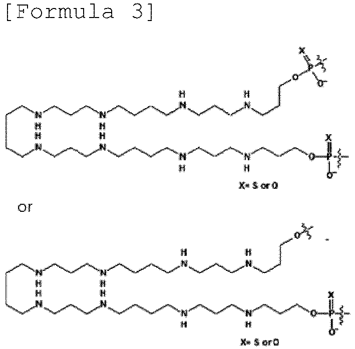

Meanwhile, the octamine derivative may be duodecamine phosphoamidite which is represented by the following Formula 4 (C₉₈H₁₂₅F₃₆N₁₄O₁₇P, molecular weight: 2486.04) .

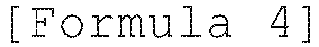

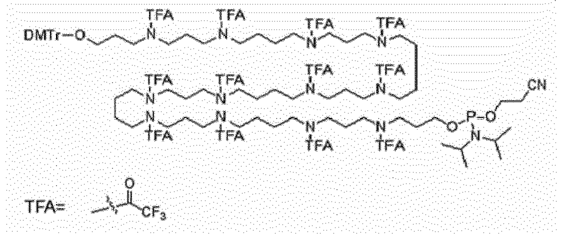

Meanwhile, the structure in which duodecamine phosphoamidite is bound to the probe is represented by the following Formula 5 (C₄₄H₁₀₀N₁₂O₂, molecular weight: 829.34) or Formula 6 (C₄₄H₁₀₀N₁₂O₄P-, molecular weight: 892.32).

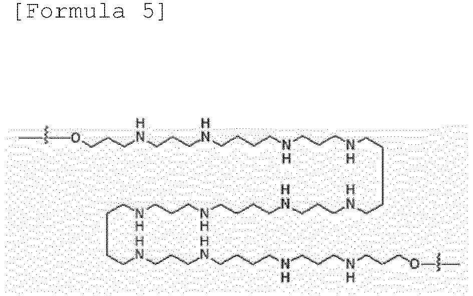

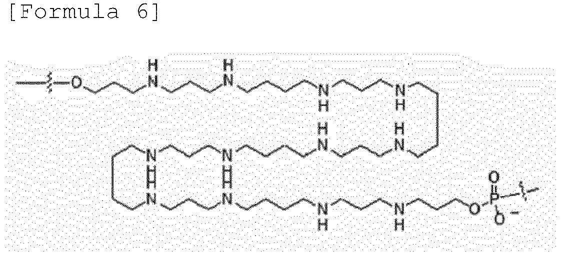

In the present invention, the octamine derivative may be characterized in that one residue of a phosphoric acid group at one end is substituted with oxygen or sulfur for binding to an amine, but is not limited thereto.

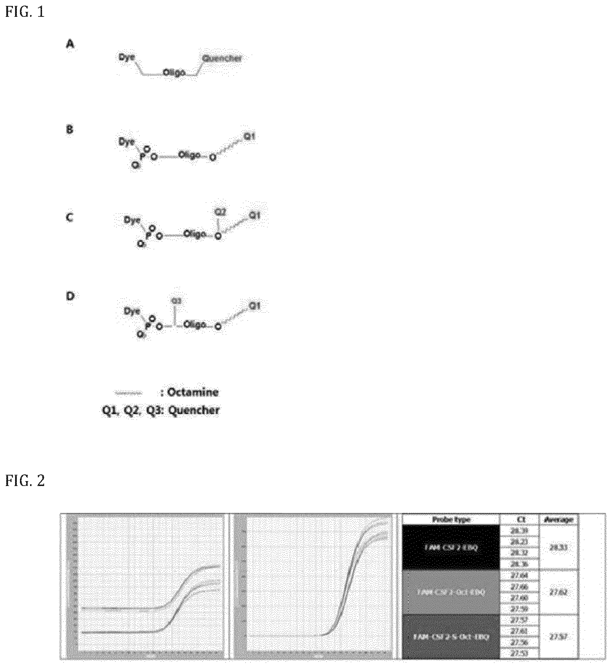

In one embodiment of the present invention, the probe may have a structure in which a reporter is bound to one end of oligonucleotide, and an octamine or octamine derivative is bound to the other end thereof, and in which a quencher is further bound to the octamine or octamine derivative. For example, the probe may have a structure in which the reporter is covalently bonded to one end of the oligonucleotide via PO₃⁻ as a linker and the octamine or octamine derivative is covalently bonded to the other end of the oligonucleotide via O as a linker, and the quencher is bound to the oligonucleotide by covalently binding to the octamine or octamine derivative (FIG. 1). Meanwhile, the octamine or octamine derivative may be bound through phosphorothioate to the end of the oligonucleotide via S as a linker.

In another aspect, the present invention is directed to a method for amplifying a target nucleic acid comprising performing a quantitative real-time polymerase chain reaction using the probe.

In the present invention, the quantitative real-time polymerase chain reaction (qPCR) may be a probe hydrolysis quantitative real-time polymerase chain reaction (probe hydrolysis qPCR).

In the present invention, the octamine or octamine derivative bound to the probe may improve the effect of suppressing light emission of the reporter by the quencher, and the effect of suppressing light emission may comprise at least one effect selected from: i) an effect of reducing basal fluorescence; ii) an effect of increasing delta fluorescence; and iii) an effect of reducing a cycle at threshold.

As used herein, the term "target nucleic acid" refers to a nucleic acid sequence to be detected, and is annealed or hybridized with a primer or probe under hybridization, annealing or amplification conditions. The term "target nucleic acid" does not differ in meaning from the term "target nucleic acid sequence" or "target sequence", and is used interchangeably therewith herein.

As used herein, the term "hybridization" refers to a phenomenon in which complementary single-stranded nucleic acids form double-stranded nucleic acids. Hybridization may occur when the complementarity between the two nucleic acid strands is perfect (perfect match), or when some mismatched bases exist. The degree of complementarity required for hybridization may vary depending on the hybridization conditions, and in particular, may be controlled by temperature.

In another aspect, the present invention is directed to a kit for quntiatative real-time polymerase chain reaction comprising the probe.

The kit used in the present invention may include a reagent containing all components necessary for amplifying a target nucleic acid, and the reagent may be provided in a ready-to-use form for real-time qualitative or quantitative detection of a target nucleic acid or RNA in a sample using a real-time nucleic acid amplifier.

The reagent in the kit used in the present invention comprises a buffer solution for reaction required for nucleic acid amplification, MgCl₂, 4 types of dNTPs, a polymerase, a primer and a detection label, and optionally contains a stabilizer, and may be present in a dried state. The detection label may be the probe of the present invention. The reagent may be provided in a dried form through freeze drying, vacuum drying, heat drying, or reduced-pressure drying.

Hereinafter, the present invention will be described in more detail with reference to examples. However, it will be obvious to those skilled in the art that these examples are provided only for illustration of the present invention and should not be construed as limiting the scope of the present invention.

### Example 1: Preparation of ethylene propylene octamine

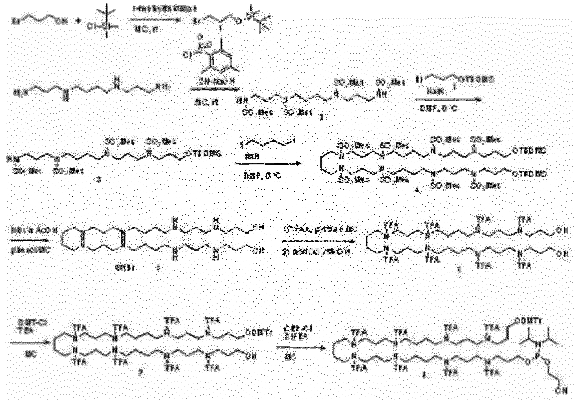

### Process 1. Substitution reaction of TBDMS (tert-butyldimethylsilyl chloride) protection group

38 g (273.4 mmol) of 3-bromo-1-propanol, 49 g (601.48 mmol) of 1-methylimidazole, and 663 g of methylene chloride were charged in a 1L round bottom flask, followed by stirring. The reaction solution was cooled to 0°C and 45.3 g (300.7 mmol) of *tert*butyldimethylsilyl chloride (TBDMSCl) was added thereto, followed by stirring for 1 day. The reaction solution was extracted in distilled water. The methylene chloride layer was collected and dried over anhydrous sodium sulfate, and the filtrate obtained by filtration was concentrated under reduced pressure. The result was separated by a column (hexane) and was concentrated under reduced pressure to obtain 59 g (85%) of the target Compound 1.

¹H NMR (300 MHz, CDCl₃) 3.76∼3.71 (t, 2H), 3.55∼3.49 (t, 2H), 2.06∼2.00 (m, 2H), 0.87 (s, 9H), 0.07 (s, 6H).

### Process 2. Substitution reaction of mesitylene sulfonyl protection group

30.0 g (148.26 mmol) of an octamine derivative, 464 g of methylene chloride, and 370 g (741.3 mmol) of a 2N aqueous sodium hydroxide solution were charged in a 1L round bottom flask, followed by stirring. The reaction solution was cooled to 0°C and a solution of 2-mesitylene sulfonyl chloride in methylene chloride was added thereto, followed by stirring at room temperature for 3 hours. The reaction solution was extracted in 722 g of ethyl acetate and 350 g of saturated sodium bicarbonate. The organic layer was collected and dried over anhydrous sodium sulfate, and the filtrate obtained by filtration was concentrated under reduced pressure. The result was separated by a column (methylene chloride: nucleic acid = 1:5) and was concentrated under reduced pressure to obtain 94.71 g (69%) of the target Compound 2.

¹H NMR (300 MHz, CDCl₃) 7.00∼6.90 (m, 8H), 4.90∼4.80 (t, 2H, NH), 3.25∼3.10 (t, 4H), 3.10∼2.95 (m, 4H), 2.85∼2.70(m, 4H), 2.60∼2.48 (m, 24H), 2.29 (s, 12H), 1.70∼1.55(m, 4H), 1.38∼1.20(m, 4H).

### Process 3. Substitution reaction of TBDMS protection group

80.98 g (86.95 mmol) of the compound of Process 2 and 274 g of dimethylformamide were charged in a 1L round bottom flask, followed by stirring. The reaction solution was cooled to 0°C and 4.5 g (113.04 mmol) of sodium hydride was slowly added thereto, followed by stirring at room temperature for 1 hour. The reaction solution was cooled to 0°C again, and a dilution of 24.2 g (95.65 mmol) of the compound of Process 1 in dimethylformamide was carefully added thereto, followed by stirring at room temperature for 1 day. The reaction solution was slowly added to 600 g of ice water while stirring, and the resulting reaction solution was concentrated under reduced pressure. The reaction solution was extracted in methylene chloride and distilled water. The organic layer was washed with brine, the methylene chloride layer was collected, dried over anhydrous sodium sulfate and filtered, and the resulting filtrate was concentrated under reduced pressure. The result was separated by a column (methylene chloride: nucleic acid = 1:5) and was concentrated under reduced pressure to obtain 43 g (36%) of the target Compound 3.

¹H NMR (300 MHz, CDCl₃) : 7.00∼6.90 (m, 8H), 5.00∼4.98 (t, 1H, NH), 3.40∼3.45 (m, 2H), 3.28∼3.18 (m, 2H), 3.10∼2.95 (m, 10 H), 2.90∼2.80 (m, 2H), 2.60∼2.50 (m, 2H), 2.29 (s, 12H), 1.70∼1.60 (m, 4H), 1.50∼1.40 (m, 2H), 1.38∼1.20 (m, 4H), 0.81 (s, 9H), -0.05 (s, 6H).

### Process 4. Octamine derivative linkage reaction

43.57 g (39.48 mmol) of the compound of Process 3 and 186 g of dimethylformamide were charged in a 1 L round bottom flask, followed by stirring. The reaction solution was cooled to 0°C and 1.65 g (41.36 mmol, 2.2 eq, 60%) of sodium hydride was added dropwise thereto, followed by stirring at room temperature for 1 hour. Then, 5.8 g (18.8 mmol, 1.0 eq) of 1,4-diodobutane was added to the reaction solution, followed by stirring at room temperature for 1 day. The reaction solution was gradually added dropwise to 300 g of ice water while stirring, and then the reaction solution was concentrated under reduced pressure. The reaction solution was extracted in methylene chloride. The methylene chloride layer was collected, dried over anhydrous sodium sulfate and filtered, and the filtrate was concentrated under reduced pressure. The result was separated by a column (nucleic acid:ethyl acetate = 3:1), and the solution was concentrated under reduced pressure to obtain 36.62 g (86%) of the target Compound 4.

¹H NMR (300 MHz, CDCl₃) : 7.00∼6.85 (m, 16H), 3.45∼3.33 (t, 4H), 3.10∼2.85 (m, 32H), 2.62∼2.48 (m, 48H), 2.35∼2.20 (m, 24H), 1.70∼1.40 (m, 12H), 1.45∼1.15 (m, 12H), 0.80 (s, 18H), -0.06 (s, 12H).

### Process 5. Reaction for removal of 2-mesitylene sulfonyl protection group

31.51 g (13.932 mmol) of the compound of Process 4 and 398 g of methylene chloride were charged in a 1 L round-bottom flask, and 102.3 g of phenol was added thereto, followed by stirring. Then, the reaction solution was cooled to 0°C, 391 g (1594.99 mmol) of a hydrobromic acid solution in acetic acid was added dropwise thereto for 1 hour, and the mixture was stirred at room temperature for 1 day. The reaction solution was added dropwise to 500 g of ice water while stirring, and was then extracted in methylene chloride. The collected water layer was concentrated under reduced pressure. 700 g of dimethyl ether was added to the result, followed by stirring for 1 hour. The resulting reaction solution was filtered, and the resulting solid compound was vacuum-dried to obtain 13.99 g of the target Compound 5.

¹H NMR (300 MHz, D₂O); 3.60∼3.40 (s, 4H), 3.20∼2.80 (m, 32H), 2.0∼1.80 (m, 12H), 1.75∼1.40 (m, 12H).

### Process 6. Substitution reaction of TFA (trifluoroacetic acid) protection group

16.99 g (13.90 mmol) of the compound of Process 5, 213 g of methylene chloride, and 78 g (1000 mmol) of pyridine were charged in a 250 ml round bottom flask, followed by stirring. The reaction solution was cooled to 0°C and 131 g (625.5 mmol) of TFAA (trifluoroacetic anhydride) was slowly added dropwise thereto, followed by stirring at room temperature for 1 day. The reaction solution was cooled to 0°C, and then distilled water was added thereto. The result was extracted in methylene chloride, and the organic layer was collected, dried over sodium sulfate, and filtered, and the filtrate was concentrated under reduced pressure. Sodium bicarbonate and 156 ml of methanol were added to the residue, the result was stirred at room temperature for 1 day, and then the solvent was concentrated under reduced pressure. The result was separated by a column (nucleic acid: ethyl acetate = 3:1), and the solution was concentrated under reduced pressure and dried in a vacuum to yield 6.32 g (34%) of the target Compound 6.

¹H NMR (300 MHz, CDCl₃) 3.80∼3.24 (m, 36 H), 2.05∼1.80 (m, 12H), 1.70∼1.50 (m, 12H).

### Process 7. Substitution reaction of DMT (4,4-dimethoxytrityl) protection group

6.71 g (5.00 mmol) of the compound of Process 6, 398 g of methylene chloride and 1.98 g (25.0 mmol) of pyridine were charged in a 500 ml round bottom flask, followed by stirring. 32 mg (0.25 mmol) of DMAP (4-dimethylaminopyridine) was added to the reaction solution, the resulting mixture was cooled to 0°C, a solution of 2.0 g (6.0 mmol) of DMTCl (4,4'-dimethoxytrityl chloride) in 66 g of methylene chloride was added dropwise thereto, and the mixture was stirred at room temperature. Distilled water was added to the reaction solution, the mixture was extracted, and the methylene chloride layer was collected, dried over sodium sulfate and filtered. The solvent was concentrated under reduced pressure and separated by a column (methylene chloride:methanol = 20:1), and the resultant solution was concentrated under reduced pressure to obtain 3.05 g (37%) of the target Compound 7.

¹H NMR (300 MHz, CDCl₃) 7.50∼7.20 (m, 9H), 6.90∼6.78 (d, 4H), 3.79 (s, 6H), 3.80∼3.02 (m, 36H), 2.02∼1.80 (m, 12H), 1.78∼1.50 (m, 12H).

### Process 8. Substitution reaction of CEP (cyanoethyl phosphoryl)

7.78 g (4.73 mmol) of the compound of Process 7, 265 g of methylene chloride and 3.06 g (23.65 mmol) of DIPEA (N,N-diisopropylethylamine) were charged in a 500 ml round-bottom flask, followed by stirring. The reaction solution was cooled to 0°C, and 3.36 g (14.19 mmol) of CEPCl (cyanoethyl phosphor chloride) was added thereto, followed by stirring at room temperature for 2 hours. Then, the reaction solution was extracted in distilled water. The methylene chloride layer was collected, dried over sodium sulfate, and filtered. The filtrate was concentrated under reduced pressure and then separated by a column (nucleic acid: ethyl acetate = 1:2), and the solution was concentrated under reduced pressure to obtain 7.27 g (83%) of the target Compound 8.

¹H NMR (300 MHz, CDCl₃) 7.50∼7.20 (m, 9H), 6.90∼6.81 (d, 4H), 3.98∼3.02 (m, 46 H), 2.70∼2.50 (m, 2H), 2.20∼1.80 (m, 12H), 1.78∼1.42 (m, 12H), 1.40∼1.02 (m, 12H). ³¹P NMR (300 MHz, CDCl₃) ; 146∼148

### Example 2: Preparation of octamine CPG

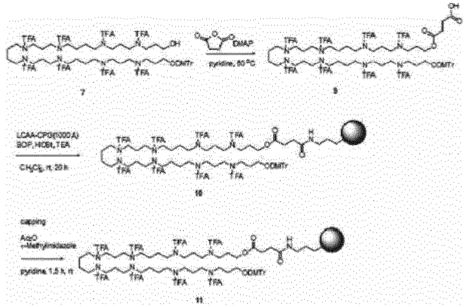

### Process 1. Succinyl substitution reaction

9.8 g of pyridine was added to 1.88 g (1.14 mmol) of Compound of Process 7 of Example 1 in a 250 ml round-bottom flask, followed by stirring. Then, 572 mg (5.71 mmol) of succinic anhydride and 69 mg (0.57 mmol) of DMAP were added to the reaction solution. The result was stirred at 60°C for 6 hours, and the solvent was concentrated under reduced pressure and extracted with methylene chloride and distilled water. The organic layer was collected, dried over sodium sulfate and filtered, and the filtrate was concentrated under reduced pressure and separated by column (methylene chloride:methanol = 20:1), and the solution was concentrated under reduced pressure to obtain 1.84 (92%) of the target Compound 9.

¹H NMR (300 MHz, CDCl) ; δ 7.42∼7.39 (m, 2H), 7.39∼7.18 (m, 7H), 6.84∼6.66 (m, 4H), 4.20∼4.16(m, 2H), 3.77 (s, 6H), 3.60∼3.02 (m, 34H), 2.80∼2.60(m, 4H), 2.02∼1.80 (m, 12H), 1.78∼1.50 (m, 12H).

### Process 2. CPG substitution reaction

1.84 g (1.05 mmol, 1.0 eq) of the compound of Process 2 of Example 1, 19 g of LCAACPG (1000Å), 697 mg (1.58 mmol) of BOP (benzotriazol-1-yloxytris(dimethylamino) phosphonium hexafluorophosphate), 213 mg (1.58 mmol) of HOBt (hydroxybenzotriazole), and 139 g of methylene chloride were charged in a 250 ml round-bottom flask, 1.1 g (11.25 mmol) of TEA (triethylamine) was added thereto, and then the mixture was allowed to stand at 30°C for 1 day. The reaction solution was filtered, washed with methylene chloride, methanol and distilled water, and dried in vacuum to yield 18.73 g of the target Compound 10.

### Process 3. Substitution reaction of acetyl protection group

18.78 g of the compound of Process 2 of Example 1 and 224 ml of pyridine were charged in a 500 ml bottle, and 20.2 g (246 mmol) of 1-methylimidazole and 25.1 g (246 mmol) of acetic anhydride were added thereto, after which the mixture was allowed to stand at room temperature for 1.5 hours. The reaction solution was filtered, washed with methylene chloride, methanol and distilled water, and dried in a vacuum to yield 18.3 g of the target Compound 11.

### Example 3: Attachment of octamine or octamine derivative to probe

The synthesis process starts from the solid support (CPG) to which the nucleoside is bound, and includes repeating a cycle consisting of deblocking, coupling, capping and oxidation. As a result, a single-stranded nucleotide having a desired sequence was obtained.

### Example 4: Comparison of CSF2 gene amplification effect between probe to which ethylene propylene octamine binds and conventional probe

A real-time gene amplification experiment was performed using a primer pair for amplifying the 143 bp sequence of the CSF gene and a probe that binds to the amplification product. The 5' end of the probe of SEQ ID NO: 3 was labeled with fluorescent substance FAM (6-carboxyfluorescein), and an EBQ quencher was bound to the 3' end thereof. Meanwhile, a FAM fluorescent substance was labeled at the 5' end of the probe of SEQ ID NO: 4 having the same nucleotide sequence, and ethylene propylene octamine and an EBQ quencher were bound to the 3' end thereof. In addition, the 5' end of the probe of SEQ ID NO: 5 having the same nucleotide sequence was labeled with a FAM fluorescent material, and the 3' end of the probe was attached with ethylene propylene octamine via phosphothioate, and an EBQ quencher was bound to the ethylene propylene octamine.

25 µl of AccuPower Plus DualStar™qPCR Master Mix (Bioneer Co., Ltd., Cat. No. K-6603), 2 µl of 10 pmol/µl of a primer pair (SEQ ID NO: 1 and SEQ ID NO: 2), and 1 µl of 10⁶ copies of CSF2 gene amplification products were mixed with 2 µl of 10 pmol/µl of a probe (SEQ ID NO: 3, SEQ ID NO: 4, or SEQ ID NO: 5), and 18 µl of distilled water was added thereto to adjust the total volume to 50 µL (Table 2). The gene amplification process was conducted by allowing the mixture to stand at 95°C for 5 minutes once, followed by repeating 45 times a cycle including allowing the mixture to stand at 95°C for 5 seconds and at 55°C for 5 seconds, and fluorescence scanning (Table 3) in a real-time gene amplification device (Bioneer Co., Ltd., *Exicycler*™ 96) .

**[Table 1]**

| No. | Name | Sequence |
|---|---|---|
| SEQ ID NO: 1 | CSF2-Forward primer | 5'-CATCTCAGAAATGTTTGACCTCCAG-3' |
| SEQ ID NO: 2 | CSF2-Reverse primer | 5'-GAGGGCAGTGCTGCTTGTAGTG-3' |
| SEQ ID NO: 3 | FAM-CSF2-EBQ | 5'-TET-AGCCGACCTGCCTACAGACCCGC-EBQ-3' |
| SEQ ID NO: 4 | FAM-CSF2-Oct-EBQ | 5'-TET-AGCCGACCTGCCTACAGACCCGC-Oct- EBQ-3' |
| SEQ ID NO: 5 | FAM-CSF2-S-Oct-EBQ | 5'-TET-AGCCGACCTGCCTACAGACCCGC-S-Oct- |
| | | EBQ-3' |

**[Table 2]**

| Composition | Volume (*µ*ℓ) |
|---|---|
| 2x mastermix (K-6603, AccuPower Plus DualStar™qPCR Master Mix(2X), 2.5ml, 100 rxn) | 25 |
| 10 pmol/*µ*ℓ, Forward primer | 2 |
| 10 pmol/*µ*ℓ Reverse primer | 2 |
| 10 pmol/*µ*ℓ Probe | 2 |
| CSF2 PCR product (10⁸ copies/µl) | 1 |
| D.W. | 18 |

**[Table 3]**

| Process | Temperature | Time | Number of repetitions |
|---|---|---|---|
| 1 | 95°C | 5 min | 1 |
| 2 | 95°C | 5 sec | 45 |
| 3 | 55°C | 5 sec | |
| 4 | Scan | | |
| 5 | 25°C | 1 min | 1 |

As a result, as can be seen from FIG. 1, in the case of CSF2 amplification, the probes of SEQ ID NO: 4 and SEQ ID NO: 5 to which ethylene propylene octamine is bound had lower basal fluorescence than the probe of SEQ ID NO: 3, which is a general probe. Specifically, the probe of SEQ ID NO: 3 exhibited a basal fluorescence of about 55k, whereas the probes of SEQ ID NO: 4 and SEQ ID NO: 5, to which ethylene propylene octamine is bound, were reduced to about 20k, corresponding to about 36% of the fluorescence of the general probe. SEQ ID NO: 3 and SEQ ID NO: 4 exhibited equivalent delta fluorescence, and SEQ ID NO: 5, containing phosphothioate, was improved by 15% compared to a general probe. The probes of SEQ ID NO: 4 and SEQ ID NO: 5 exhibited a decreased cycle at threshold (Ct) of about 0.71 and about 0.76, respectively, compared to the probe of SEQ ID NO: 3. The result showed that ethylene propylene octamine improved the efficiency of suppression on emission of the FAM fluorescent material by EBQ, and decreased the cycle at threshold (Ct) as well. In particular, the probe in which ethylene propylene octamine was bound through phosphothioate to the 3' end thereof and to which an EBQ quencher was bound exhibited improved delta fluorescence and improved efficiency of suppression of emission of the FAM fluorescent material by EBQ, and thus reduced the cycle at threshold (Ct) compared to the probe in which ethylene propylene octamine was bound to the 3' end thereof without phosphothioate and to which an EBQ quencher was bound.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Industrial applicability]

When the probe according to the present invention is used, the octamine or octamine derivative bound to the probe enables the quencher to effectively suppresses the emission of the reporter, thus exhibiting effects such as i) an effect of reducing basal fluorescence, ii) an effect of increasing delta fluorescence, and iii) an effect of decreasing a cycle at threshold, thereby being useful for various quantitative real-time polymerase chain reactions requiring accuracy and sensitivity.

### [Sequence Free Text]

An electronic file is attached.

## Claims

1. A probe to which a reporter, a quencher, and an octamine or octamine derivative are bound.

2. The probe according to claim 1, wherein the probe is selected from the group consisting of synthetic artificial deoxyribonucleic acid (DNA), peptide nucleic acid (PNA), and locked nucleic acid (LNA).

3. The probe according to claim 1, wherein the reporter is at least one selected from the group consisting of FAM (6-carboxyfluorescein), TET (5-tetrachlorofluorescein), Texas red, HEX (2',4',5',7',-tetrachloro-6-carboxy-4,7-dichlorofluorescein), Joh, Cy3, Rox, Cy5.5 and CY5.

4. The probe according to claim 1, wherein the quencher is at least one selected from the group consisting of 6-carboxytetramethyl-rhodamine (TAMRA), BHQ1, BHQ2, EBQ and Dabcyl.

5. The probe according to claim 1, wherein the octamine is ethylene propylene octamine.

6. The probe according to claim 1, wherein the octamine derivative is duodecamine phosphoamidite.

7. The probe according to claim 1, wherein the probe has a structure in which the reporter is bound to one end of an oligonucleotide, the octamine or octamine derivative is bound to the other end thereof, and the quencher is further bound to the octamine or octamine derivative.

8. The probe according to claim 7, wherein the octamine or octamine derivative is bound through phosphorothioate to the other end of the oligonucleotide.

9. A method for amplifying a target nucleic acid comprising performing a quantitative real-time polymerase chain reaction using the probe according to any one of claims 1 to 8.

10. The method according to claim 9, wherein the quantitative real-time polymerase chain reaction (qPCR) is a probe hydrolysis-based quantitative real-time polymerase chain reaction (probe hydrolysis qPCR).

11. The method according to claim 9, wherein the octamine or octamine derivative bound to the probe improves an effect of suppressing light emission of the reporter by the quencher.

12. The method according to claim 11, wherein the effect of suppressing light emission comprises at least one selected from the following effects:
i) an effect of reducing basal fluorescence;
ii) an effect of increasing delta fluorescence; and
iii) an effect of reducing a cycle at threshold.

13. A kit for quantitative real-time polymerase chain reaction comprising the probe according to any one of claims 1 to 8.


<110>     BIONEER CORPORATION

<120>     A Probe linked with Octamine or Octamine Derivatives and Use of
          the Same

<130>     PP-B2292

<150>     KR 10-2018-0125973
<151>     2018-10-22

<160>     5

<170>     KoPatentIn 3.0

<210>     1
<211>     25
<212>     DNA
<213>     Artificial Sequence

<220>
<223>     CSF2-Forward primer


<400>     1
catctcagaa atgtttgacc tccag                                              25


<210>     2
<211>     22
<212>     DNA
<213>     Artificial Sequence

<220>
<223>     CSF2-Reverse primer


<400>     2
gagggcagtg ctgcttgtag tg                                                 22


<210>     3
<211>     23
<212>     DNA
<213>     Artificial Sequence

<220>
<223>     FAM-CSF2-EBQ


<400>     3
agccgacctg cctacagacc cgc                                                23


<210>     4
<211>     23
<212>     DNA
<213>     Artificial Sequence

<220>
<223>     FAM-CSF2-Oct-EBQ


<400>     4

agccgacctg cctacagacc cgc                                                23

<210>    5
<211>    23
<212>    DNA
<213>    Artificial Sequence

<220>
<223>    FAM-CSF2-S-Oct-EBQ

<400>    5
agccgacctg cctacagacc cgc                                                23